(19) Europäisches Patentamt
European Patent Office
Office européen des brevets

(11) Veröffentlichungsnummer: **0 081 142**
**B1**

(12) # EUROPÄISCHE PATENTSCHRIFT

(45) Veröffentlichungstag der Patentschrift: **25.06.86**

(51) Int. Cl.[4]: **C 07 D 251/34,** A 01 N 43/64

(21) Anmeldenummer: **82110859.4**

(22) Anmeldetag: **24.11.82**

(54) **1,3,5-Triazinone, Verfahren zu ihrer Herstellung und ihre Verwendung zur Bekämpfung unerwünschten Pflanzenwuchses.**

(30) Priorität: **03.12.81 DE 3147879**
**16.01.82 DE 3201229**

(43) Veröffentlichungstag der Anmeldung:
**15.06.83 Patentblatt 83/24**

(45) Bekanntmachung des Hinweises auf die Patenterteilung:
**25.06.86 Patentblatt 86/26**

(84) Benannte Vertragsstaaten:
**AT BE CH DE FR GB IT LI NL SE**

(56) Entgegenhaltungen:
**DE - A - 2 246 109**
**FR - A - 2 388 559**

(73) Patentinhaber: **BASF Aktiengesellschaft,**
**Carl-Bosch-Strasse 38, D-6700 Ludwigshafen (DE)**

(72) Erfinder: **Parg, Adolf, Dr., Paray-le-Monial-Strasse 8,**
**D-6702 Bad Duerkheim (DE)**
Erfinder: **Hamprecht, Gerhard, Dr.,**
**Rote-Turm-Strasse 28, D-6940 Weinheim (DE)**
Erfinder: **Wuerzer, Bruno, Dr. Dipl.-Landwirt,**
**Ruedigerstrasse 13, D-6701 Otterstadt (DE)**

**EP 0 081 142 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft 1,3,5-Triazinone, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Es ist bekannt, phenoxysubstituierte N-Phenyl-1,3,5-triazinone, wie
1-[4-(2'-Chlor-4'-trifluormethylphenoxy)-phenyl]-3-methyl-1,3,5-triazin-2,4,6-(1H,3H,5H)-trion,
als Arzneimittel, insbesondere als Coccidiostatica, zu verwenden (DE-OS 2 246 109).

Es wurde gefunden, dass 1,3,5-Triazinone der Formel

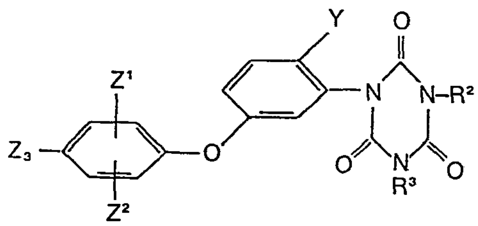

(I),

in der

$Z^1$ und $Z^2$ jeweils unabhängig voneinander Wasserstoff, Halogen, Nitro, Cyano, Carboxyl, Alkyl, Halogenalkyl oder Alkoxy mit jeweils bis zu 4 Kohlenstoffatomen,

$Z^3$ Halogen, Nitro, Cyano, Alkyl, Halogenalkyl, Alkoxy, Halogenalkoxy, Alkylmercapto, Halogenalkylmercapto, Alkylsulfinyl, Halogenalkylsulfinyl, Alkylsulfonyl oder Halogenalkylsulfonyl mit jeweils bis zu 4 Kohlenstoffatomen,

Y Wasserstoff, Halogen, Cyano oder Nitro,

$R^2$ Wasserstoff, einen gesättigten oder ungesättigten, unverzweigten oder verzweigten aliphatischen Kohlenwasserstoffrest mit bis zu 20 Kohlenstoffatomen, einen durch Halogen, Cyano, Hydroxy, Mercapto, Alkoxy oder Alkylmercapto mit jeweils bis zu 4 Kohlenstoffatomen, Phenylmercapto, Alkyl- oder Dialkylamino mit 1 bis 4 Kohlenstoffatomen in einer Alkylgruppe substituierten gesättigten, unverzweigten oder verzweigten aliphatischen Kohlenwasserstoffrest mit bis zu 10 Kohlenstoffatomen, einen gegebenenfalls durch Alkoxy mit 1 bis 4 Kohlenstoffstomen substituierten Cycloalkylrest mit 3 bis 7 Kohlenstoffatomen, einen gegebenenfalls durch Halogen, Alkyl oder Alkoxy mit jeweils 1 bis 4 Kohlenstoffatomen, Nitro, Cyano, Halogenalkyl, Halogenalkoxy oder Halogenalkylmercapto mit jeweils 1 bis 4 Kohlenstoffatomen substituierten Phenylrest oder einen gegebenenfalls durch Halogen substituierten Benzylrest und

$R^3$ Wasserstoff, Alkyl mit bis zu 4 Kohlenstoffatomen, gegebenenfalls halogensubstituiertes Acyl mit bis zu 7 Kohlenstoffatomen oder – bei einfach negativ geladenem zugehörigem Triazinon-Stickstoffatom – ein Alkalimetallion oder ein gegebenenfalls alkyliertes Ammoniumion

bedeuten, sehr gute herbizide und gegenüber Kulturpflanzen selektive Eigenschaften haben.

In der Formel I können $Z^1$ und $Z^2$ jeweils unabhängig voneinander beispielsweise Wasserstoff, Halogen, wie Fluor, Chlor, Brom, Jod, Nitro, Cyano, Carboxyl, Alkyl, Halogenalkyl oder Alkoxy mit jeweils bis zu 4 Kohlenstoffatomen, wie
Methyl, Ethyl, n-Propyl, i-Propyl, n-Butyl, tert.-Butyl, Trifluormethyl, Difluormethyl, Fluormethyl, Trichlormethyl, Dichlormethyl, Chlormethyl, Difluorchlormethyl, 1-Chlorethyl, 2-Chlorethyl, 1-Fluorethyl, 2-Fluorethyl, 2,2,2-Trichlorethyl, 2,2,2-Trifluorethyl, 1,1,2,2-Tetrafluorethyl, 1,1,2-Trifluor-2-chlorethyl, 1,1,2,2,2-Pentafluorethyl, Methoxy, Ethoxy, n-Propyloxy, i-Propyloxy oder tert.-Butyloxy, und

$Z^3$ Halogen, wie Fluor, Chlor, Brom, Jod, Nitro, Cyano, Alkyl, Halogenalkyl, Alkoxy, Halogenalkoxy, Alkylmercapto, Halogenalkylmercapto, Alkylsulfinyl, Halogenalkylsulfinyl, Alkylsulfonyl oder Halogenalkylsulfonyl mit jeweils bis zu 4 Kohlenstoffatomen, wie
Methyl, Ethyl, n-Propyl, i-Propyl, n-Butyl, tert.-Butyl, Trifluormethyl, Difluormethyl, Fluormethyl, Trichlormethyl, Dichlormethyl, Chlormethyl, Difluorchlormethyl, 1-Chlorethyl, 2-Chlorethyl, 1-Fluorethyl, 2-Fluorethyl, 2,2,2-Trichlorethyl, 2,2,2-Trifluorethyl, 1,1,2,2-Tetrafluorethyl, 1,1,2-Trifluor-2-chlorethyl, 1,1,2,2,2-Pentafluorethyl, Methoxy, Ethoxy, n-Propyloxy, i-Propyloxy, tert.-Butyloxy, Trichlormethoxy, Trifluormethoxy, 1-Chlorethoxy, 2-Chlorethoxy, 1-Fluorethoxy, 2-Fluorethoxy, 2,2,2-Trichlorethoxy, 2,2,2-Trifluorethoxy, 1,1,2,2-Tetrafluorethoxy, 1,1,2,2,2-Pentafluorethoxy, Methylmercapto, Ethylmercapto, Trichlormethylmercapto, Trifluormethylmercapto, Methylsulfinyl, Ethylsulfinyl, Methylsulfonyl, Ethylsulfonyl oder Trifluormethylsulfonyl
bedeuten.

Y kann beispielsweise für Halogen, wie Fluor, Chlor, Brom, Jod sowie für Wasserstoff, Cyano oder Nitro stehen.

$R^2$ in Formel I steht für Wasserstoff, einen gesättigten oder ungesättigten, unverzweigten oder verzweigten aliphatischen Kohlenwasserstoffrest mit bis zu 20 Kohlenstoffatomen, beispielsweise für einen Alkyrest mit bis zu 20 Kohlenstoffatomen, vorzugsweise mit bis zu 12, insbesondere mit bis zu 4 Kohlenstoffatomen, für einen Alkenyl- oder Alkinylrest mit bis zu 20 Kohlenstoffatomen, vorzugsweise mit bis zu 12, insbesondere mit bis zu 4 Kohlenstoffatomen, wie
Methyl, Ethyl, n-Propyl, Isopropyl, n-Butyl, Isobutyl, sec.-Butyl. tert.-Butyl, n-Pentyl, tert. Amyl, n-Hexyl, Pentyl-3, 1,2-Dimethyl-n-propyl, 1,3-Dimethyl-n-butyl, 1-Ethyl-2-methyl-n-propyl, 1,2,2-Trimethyl-n-propyl, 1,2-Dimethyl-4-hexyl, Allyl, Methallyl, Crotyl, 2-Ethyl-hex-2-enyl, Hex-5-enyl, 2-Methyl-but-2-enyl, 2-Methyl-but-3-enyl,

But-1-en-3-yl, 2-Methyl-but-1-en-4-yl,
2-Methyl-but-2-en-4-yl,
3-Methyl-but-1-en-3-yl, Propargyl,
But-1-in-3-yl, But-2-inyl,
für einen durch Halogen, Cyano, Hydroxy, Mercapto oder Alkoxy mit 1 bis 4 Kohlenstoffatomen substituierten gesättigten, unverzweigten oder verzweigten aliphatischen Kohlenwasserstoffrest mit bis zu 10 Kohlenstoffatomen, vorzugsweise mit bis zu 4 Kohlenstoffatomen, beispielsweise einen durch Halogen, Cyano, Hydroxy, Mercapto oder Alkoxy mit jeweils bis zu 4 Kohlenstoffatomen substituierten Alkylrest mit bis zu 10 Kohlenstoffatomen, vorzugsweise mit 1 bis 4 Kohlenstoffatomen, wie
2-Chlorethyl, 2-Chlor-n-propyl,
3-Chlor-n-propyl, 2-Chlor-sec.-butyl,
2-Chlor-isobutyl, 2-Fluor-sec.-butyl,
2-Fluor-isobutyl, 2-Fluor-isopropyl,
Chlor-tert.-butyl, 2,2,2-Trifluorethyl,
1-Cyanomethyl, 2-Cyanomethyl, 2-Hydroxyethyl,
3-Hydroxy-n-propyl, 2-Mercapto-ethyl,
3-Mercapto-n-propyl, 2-Methoxyethyl,
2-Ethoxyethyl, 3-Methoxy-n-propyl,
2-Methoxy-isopropyl, 3-Methoxy-n-butyl,
1-Methoxy-sec.-butyl, Methoxy-tert.-butyl,
Ethoxy-tert.-butyl, 2-Methoxy-n-butyl,
4-Methoxy-n-butyl,
oder für einen gegebenenfalls durch Alkoxy mit 1 bis 4 Kohlenstoffatomen substituierten Cycloalkylrest mit 3 bis 7 Kohlenstoffatomen, wie Cyclopropyl, Cyclopentyl, Cyclohexyl, 4-Ethoxycyclohexyl.

$R^2$ steht ausserdem für einen durch Phenylmercapto, Alkylmercapto mit bis zu 4 Kohlenstoffatomen oder Alkyl- oder Dialkylamino mit 1 bis 4 Kohlenstoffatomen in einer Alkylgruppe substituierten gesättigten, unverzweigten oder verzweigten aliphatischen Kohlenwasserstoffrest mit bis zu 10 Kohlenstoffatomen, beispielsweise einen durch Phenylmercapto oder Alkylmercapto mit 1 bis 4 Kohlenstoffatomen oder Alkyl- oder Dialkylamino mit 1 bis 4 Kohlenstoffatomen in einer Alkylgruppe substituierten Alkylrest mit bis zu 10 Kohlenstoffatomen, vorzugsweise mit 1 bis 4 Kohlenstoffatomen, wie
2-Methylmercapto-ethyl, 2-Ethylmercapto-ethyl,
3-Methylmercapto-n-propyl,
3-Methylmercapto-n-butyl,
1-Methylmercapto-sec.-butyl,
Methylmercapto-tert.-butyl,
2-Methylmercapto-n-butyl, 2-Methylaminoethyl,
2-Ethylaminoethyl, 2-Dimethylaminoethyl,
2-Diethylaminoethyl, 2-Dimethylamino-n-propyl,
3-Dimethylamino-n-propyl,
4-Dimethylamino-n-butyl,
oder für einen gegebenenfalls durch Halogen, Alkyl oder Alkoxy mit 1 bis 4 Kohlenstoffatomen, Nitro, Cyano, Halogenalkyl, Halogenalkoxy oder Halogenalkylmercapto mit 1 bis 4 Kohlenstoffatomen substituierten Phenylrest oder einen gegebenenfalls durch Halogen substituierten Benzylrest, wie
Phenyl, 4-Chlorphenyl, 3,4-Dichlorphenyl,
o-, m-, p-tert.-Butylphenyl,
o-, m-, p-Methoxyphenyl, o-, m-, p-Methylphenyl,
4-Methoxy-3-chlorphenyl,
2-Methyl-4-chlor-phenyl, 4-Nitrophenyl,
4-Nitro-2-chlorphenyl, o-, m-, p-Cyanophenyl,
o-, m-Trifluormethylphenyl,
2-Chlor-4-trifluormethylphenyl,
4-Trifluormethoxyphenyl,
4-Trifluormethylmercaptophenyl,
3-Trifluormethylmercaptophenyl, Benzyl,
2,6-Dichlorbenzyl, 2-Chlor-6-fluorbenzyl,
2,6-Difluor-benzyl, o-, m-, p-Chlorbenzyl.

$R^3$ kann Wasserstoff, Alkyl mit bis zu 4 Kohlenstoffatomen oder gegebenenfalls halogensubstituiertes Acyl mit bis zu 7 Kohlenstoffatomen wie Methyl, Ethyl, n-Propyl, Isopropyl, n-Butyl, 2-Butyl, Formyl, Acetyl, Chloracetyl, Benzoyl, oder – bei einfach negativ geladenem zugehörigem Triazinon-Stickstoffatom – ein Alkalimetallion oder ein gegebenenfalls alkyliertes Ammoniumion, wie Natrium, Kalium, Ammonium, Methylammonium, Dimethylammonium, Trimethylammonium oder Tetramethylammonium bedeuten.

Bevorzugte 1,3,5-Triazinone sind Verbindungen der Formel I, in der $Z^1$ und $Z^2$ jeweils unabhängig voneinander Wasserstoff, Chlor, Brom oder Cyano, $Z^3$ Chlor, Brom, Methyl, Trifluormethoxy, Trifluormethylmercapto oder Trifluormethyl, vorzugsweise Trifluormethyl, Y Brom oder Nitro, insbesondere Nitro, $R^2$ Alkyl mit 1 bis 4 Kohlenstoffatomen, durch Halogen, Cyano, Alkoxy oder Alkylmercapto substituiertes Alkyl mit bis zu 4 Kohlenstoffatomen, Cycloalkyl mit 3 bis 7 Kohlenstoffatomen, durch Halogenalkyl mit 1 bis 4 Kohlenstoffatomen oder Halogen substituiertes Phenyl oder durch Halogen substituiertes Benzyl, vorzugsweise
Methyl, Ethyl, 2-Chlorethyl, 2-Cyanoethyl,
2-Methoxyethyl, 2-Methylmercaptoethyl,
Cyclohexyl, 3,4-Dichlorphenyl,
3-Trifluormethylphenyl oder 4-Chlorbenzyl,
insbesondere Methyl, 2-Chlorethyl,
2-Methoxyethyl oder 3,4-Dichlorphenyl, und
$R^3$ Wasserstoff, Methyl oder Natrium
bedeuten.

Die Verbindungen der Formel I mit $R^3$ = Wasserstoff können beispielsweise nach folgendem Verfahren hergestellt werden:

Man setzt den phenoxysubstituierten Harnstoff der Formel

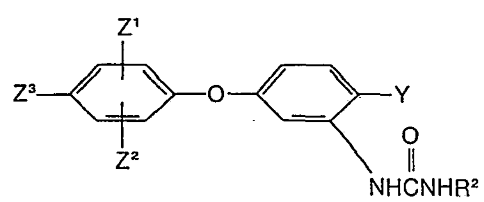
(II),

in der $Z^1$, $Z^2$, $Z^3$, Y und $R^2$ die oben genannten Bedeutungen haben, mit einem substituierten Carbonylisocyanat der Formel

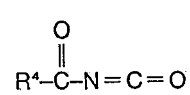
(III),

in der $R^4$ für Halogen, eine Alkoxygruppe oder eine Aryloxygruppe steht, in einem inerten organischen Lösungsmittel, gegebenenfalls unter Zusatz eines Säureacceptors, bei Temperaturen zwischen −20 und bis +180 °C, vorzugsweise zwischen +20 und +150 °C, drucklos oder unter Druck, kontinuierlich oder diskontinuierlich, zu einem substituierten 1,3,5-Triazinon der Formel

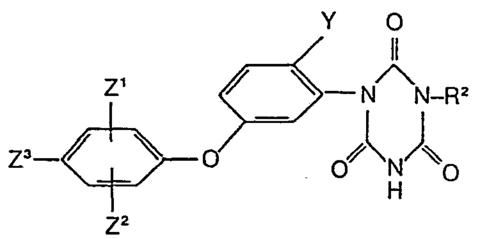
,

in der $Z^1$, $Z^2$, $Z^3$, Y und $R^2$ die obengenannten Bedeutungen haben, um.

Dieses kann dann gegebenenfalls mit einem Acylhalogenid der Formel $R^3COX$ oder einem Alkylhalogenid der Formel $R^3X$ oder einem Dialkylsulfat der Formel $(R^3O)_2SO_2$, wobei $R^3$ jeweils die obengenannten Bedeutungen, ausgenommen Wasserstoff, hat und X für Halogen steht, acyliert oder alkyliert oder gegebenenfalls mit einem Alkalialkoholat, einem Alkalihydroxid oder einem gegebenenfalls alkylierten Ammoniumhydroxid in ein Salz der Formel I überführt werden.

Verwendet man N-3-(2'-Chlor-4'-trifluormethylphenoxy)-6-nitro-phenyl-N'-methylharnstoff und Chlorcarbonylisocyanat als Ausgangsstoffe sowie Dimethylsulfat als Alkylierungsmittel, so kann der Reaktionsablauf durch folgendes Formelschema wiedergegeben werden:

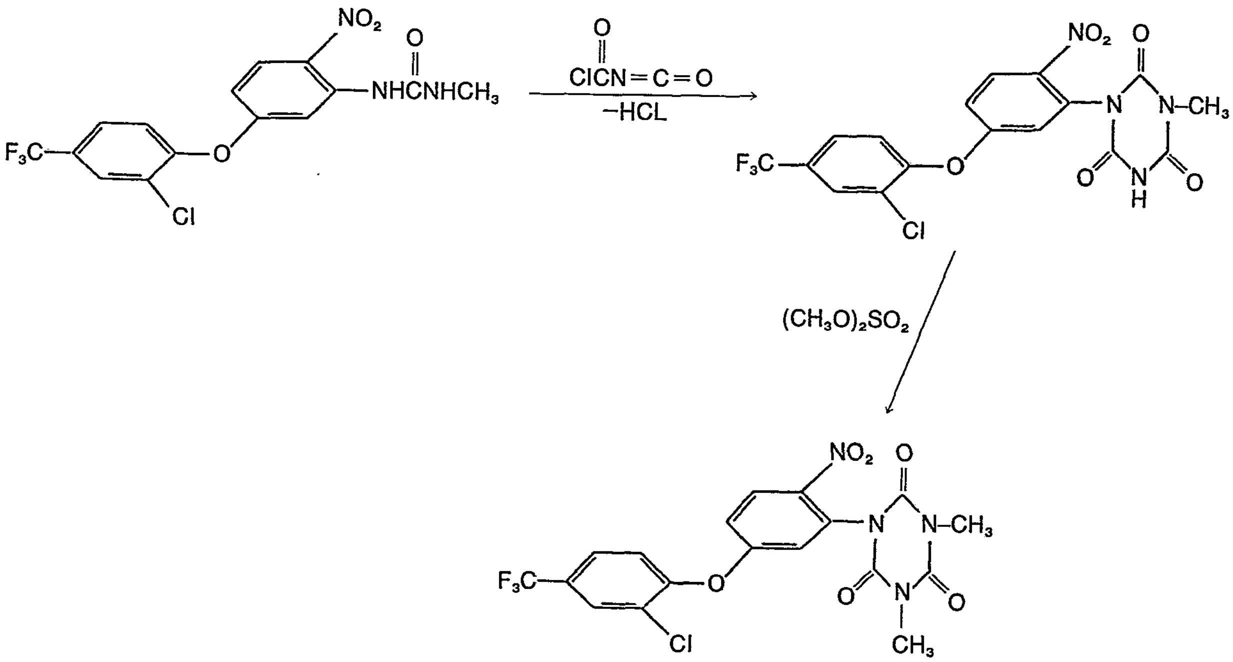


Man verwendet für die Umsetzung unter den jeweiligen Reaktionsbedingungen inerte organische Lösungsmittel. Als Lösungsmittel kommen z. B. in Betracht: Halogenkohlenwasserstoffe, insbesondere aromatische oder aliphatische Chlorkohlenstoffwasserstoffe, z. B.
Tetrachlorethylen,
1,1,2,2- oder 1,1,1,2-Tetrachlorethan,
Dichlorpropan, Methylenchlorid, Dichlorbutan,
Chloroform, Chlornaphthalin, Dichlornaphthalin,
Tetrachlorkohlenstoff,
1,1,1- oder 1,1-2-Trichlorethan,
Trichlorethylen, Pentachlorethan,
o-, m-, p-Difluorbenzol, 1,2-Dichlorethan,
1,1-Dichlorethan, 1,2-cis-Dichlorethylen,
Chlorbenzol, Fluorbenzol, Brombenzol,
Jodbenzol, o-, m-, p-Dichlorbenzol,
o-, p-, m-Dibrombenzol, o-, m-, p-Chlortoluol,
1,2,4-Trichlorbenzol;
Ether, z. B.
Ethylpropylether, Methyl-tert.-butylether,
n-Butylethylether, Di-n-butylether,
Diisobutylether, Diisoamylether,
Diisopropylether, Anisol, Phenetol,
Cyclohexylmethylether, Diethylether,
Ethylenglykoldimethylether, Tetrahydrofuran,
Dioxan, β,β'-Dichlordiethylether;
Nitrokohlenwasserstoffe, wie
Nitromethan, Nitroethan, Nitrobenzol,
o-, m-, p-Chlornitrobenzol, o-Nitrotoluol;
Nitrile, wie
Acetonitril, Butyronitril, Isobutyronitril,
Benzonitril, m-Chlorbenzonitril;
aliphatische oder cycloaliphatische Kohlenwasserstoffe, z. B. Heptan, Pinan, Nonan, o-, m-, p-Cymol, Benzinfraktionen innerhalb eines Siedepunktintervalles von 70 bis 190 °C,
Cyclohexan, Methylcyclohexan, Dekalin,
Petrolether, Hexan, Ligroin,
2,2,4-Trimethylpentan, 2,2,3-Trimethylpentan,
2,3,3-Trimethylpentan, Octan;
Ester, z. B.
Ethylacetat, Acetessigester, Isobutylacetat;
Amide, z. B.

Formamid, Methylformamid, Dimethylformamid;
Ketone, z.B.
Aceton, Methylethylketon,
und entsprechende Gemische. Zweckmässigerweise verwendet man das Lösungsmittel in einer Menge von 100 bis 2000 Gew.%, vorzugsweise von 200 bis 700 Gew,%, bezogen auf die Ausgangsstoffe.

Die bei der Reaktion entstehende Salzsäure entweicht gasförmig oder wird durch Säureacceptoren gebunden. Als Säureacceptoren können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Es kommen z.B. folgende basische Verbindungen in Frage:
Kaliumhydroxid, Natriumcarbonat,
Lithiumhydroxid, Lithiumcarbonat,
Natriumbicarbonat, Kaliumbicarbonat,
Calciumhydroxid, Calciumoxid, Bariumoxid,
Magnesiumhydroxid, Magnesiumoxid,
Bariumhydroxid, Calciumcarbonat,
Magnesiumcarbonat, Magnesiumbicarbonat,
Magnesiumacetat, Zinkhydroxid, Zinkoxid,
Zinkcarbonat, Zinkbicarbonat, Zinkacetat,
Natriumformiat, Natriumacetat, Trimethylamin,
Triethylamin, Tripropylamin, Triisopropylamin,
Tributylamin, Triisobutylamin,
Tri-sec-butylamin, Tri-tert.-butylamin,
Tribenzylamin, Tricyclohexylamin, Triamylamin,
Diisopropylethylain, Trihexylamin,
N,N-Dimethylanilin, N,N-Diethylanilin,
N,N-Dipropyltoluidin,
N,N-Dimethyl-p-aminopyridin,
N-Methylpyrrolidon, N-Ethylpyrrolidon,
N-Methylpiperidin, N-Ethylpiperidin,
N-Methylpyrrolidin, N-Ethylpyrrolidin,
N-Methylimidazol, N-Ethylimidazol,
N-Methylpyrrol, N-Ethylpyrrol,
N-Methylmorpholin, N-Ethylmorpholin,
N-Methylhexamethylenimin,
N-Ethylhexamethylenimin, Pyridin, Chinolin,
-Picolin, β-Picolin, -Picolin, Isochinolin,
Pyrimidin, Acridin,
N,N,N',N'-Tetramethylethylendiamin,
N,N,N',N'-Tetraethylethylendiamin,
Chinoxalin, Chinazolin,
N-Propyldiisopropylamin,
N,N-Dimethylcyclohexylamin,
2,6-Lutidin, 2,4-Lutidin, Trifurfurylamin,
Triethylendiamin.

Die Ausgangsstoffe werden z.B. in ungefähr stöchiometrischem Verhältnis zur Reaktion gebrqcht, d.h. Ausgangsstoff III kann z.B. in einem Überschuss bis zu 20% (Mol%), bezogen auf II, eingesetzt werden.

Zweckmässigerweise wird das Verfahren zur Herstellung der Verbindungen der Formel I so durchgeführt, dass man den Ausgangsstoff II, gegebenenfalls in einem der vorgenannten Verdünnungsmittel, vorlegt und dann den Ausgangsstoff III und gegebenenfalls einen Säureakzeptor gleichzeitig oder nacheinander zugibt. Man kann jedoch auch den Ausgangsstoff III in einem Verdünnungsmittel vorlegen und dann den Ausgangsstoff II und einen Säureakzeptor, gleichzeitig oder in beliebiger Reihenfolge, über zwei getrennte Zuführungen zugeben.

Die Umsetzung ist in vielen Fällen nach der Zugabe der Komponenten bereits abgeschlossen, andernfalls rührt man zu ihrer Beendigung noch 10 Minuten bis 10 Stunden bei −20 bis 180 °C, vorzugsweise 20 bis 150 °C, insbesondere 40 bis 100 °C, nach.

Verwendet man ein Inertgas zur Entfernung des Halogenwasserstoffes, so rührt man zweckmässigerweise 0,2 bis 10 Stunden bei 40 bis 100 °C nach.

Aus dem Reaktionsgemisch wird der Endstoff I in üblicher Weise, z.B. nach Abdestillieren von Lösungsmittel oder überschüssigem Ausgangsstoff III oder direkt durch Absaugen, isoliert. Der verbleibende Rückstand wird in diesem Fall zur Entfernung saurer Verunreinigungen mit Wasser bzw. verdünntem Alkali gewaschen und getrocknet. Im Falle von mit Wasser nicht mischbaren Verdünnungsmitteln kann man auch direkt das Reaktionsgemisch mit Wasser bzw. mit verdünntem Alkali extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation gereinigt werden.

Die Verbindungen der Formel I mit $R^3$ = Alkyl, Acyl, Alkalimetallion oder gegebenenfalls alkyliertes Ammoniumion können aus den entsprechenden Verbindungen der Formel I mit $R^3$ = Wasserstoff in an sich bekannter Weise hergestellt werden. Die Alkylierung erfolgt mittels Alkylierungsagentien, wie Alkylhalogeniden (z.B. Methylbromid, Ethyljodid), Dialkylsulfaten (z.B. Dimethylsulfat, Diethylsulfat) oder Oxomiumsalzen (z.B. Trimethyloxoniumtetrafluorborat) gegebenenfalls in Gegenwart eines inerten organischen Lösungsmittels und gegebenenfalls in Gegenwart eines Säureakzeptors bei −20 °C bis 100 °C, vorzugsweise bei 0 bis 100 °C, drucklos oder unter Druck, kontinuierlich oder diskontinuierlich. Die Acylierung erfolgt mittels Acylhalogeniden (z.B. Acetylchlorid, Benzylchlorid) gegebenenfalls in Gegenwart eines inerten organischen Lösungsmittels und gegebenenfalls in Gegenwart eines Säureakzeptors bei −20 °C bis 150 °C, vorzugsweise 20 bis 120 °C, drucklos oder unter Druck, kontinuierlich oder diskontinuierlich.

Zur Herstellung der Salze löst man zweckmässigerweise die Verbindungen der Formel I, in denen $R^3$ Wasserstoff ist, in einem organischen Lösungsmittel, z.B. Methanol, versetzt mit der ungefähr stöchiometrischen Menge Alkalialkoholat, z.B. Natriummethylat, Alkalihydroxid, z.B. Natriumhydroxid, oder einem gegebenenfalls alkylierten Ammoniumhydroxid, z.B. Ammoniumhydroxid und engt zur Trockene ein.

Die Ausgangsverbindungen können nach bekannten Methoden hergestellt werden. So erfolgt

die Herstellung der phenoxysubstituierten Harnstoffe der Formel II beispielsweise nach der in der DE-OS 2 942 930 beschriebenen Verfahrensweise. Die Verbindungen der Formel III können nach literaturbekannten Methoden hergestellt werden (Angew. Chem. 89 (1977), 789).

Die folgenden Beispiele erläutern die Herstellung der Verbindungen der Formel I nach dem angegebenen Verfahren. Gewichtsteile verhalten sich zu Volumenteilen wie kg zu l.

Beispiel 1

Zu einer Suspension von 19,5 Gewichtsteilen N-3-(2′-Chlor-4′-trifluormethylphenoxy)-6-nitrophenyl-N′-methylharnstoff in 25 Volumenteilen absolutem Toluol fügt man eine Lösung von 6,4 Gewichtsteilen N-Chlorcarbonylisocyanat in 5 Volumenteilen absolutem Toluol zu. Die Reaktionsmischung wird erhitzt und zwei Stunden bei Rückflusstemperatur nachgerührt. Nach dem Abkühlen versetzt man die Reaktionslösung mit n-Pentan und saugt den gebildeten Niederschlag ab. Man erhält 19 Gewichtsteile (83% d.Th.) 1-[3′-(2″-Chlor-4″-trifluormethylphenoxy)-6′-nitrophenyl]-3-methyl-1,3,5-triazin-2,4,6-(1H,3H,5H)-trion (Verbindung Nr. 1) vom Schmelzpunkt 208 bis 211 °C.

Beispiel 2

Eine Lösung von 8 Gewichtsteilen der Verbindung Nr. 1 in 100 Volumenteilen Aceton wird zusammen mit 2,4 Gewichtsteilen Kaliumcarbonat und 2,2 Gewichtsteilen Dimethylsulfat zwei Stunden bei Rückflusstemperatur gerührt. Die Reaktionsmischung wird abfiltriert, unter vermindertem Druck eingedampft, und der Rückstand wird aus Diisopropylether umkristallisiert. Man erhält 8 Gewichtsteile (97% d.Th) 1-[3′-(2″-Chlor-4″-trifluormethylphenoxy)-6′-nitrophenyl]-3,5-dimethyl-1,3,5-triazin-2,4,6-(1H,3H,5H)-trion (Verbindung Nr. 2) vom Schmelzpunkt 200 bis 205 °C.

Beispiel 3

5 Gewichtsteile der Verbindung Nr. 1 werden in 50 Volumenteilen absolutem Methanol suspendiert, mit 1,96 Gewichtsteilen einer 30%igen methanolischen Natriummethylatlösung versetzt. Das Gemisch wird 30 Minuten bei Raumtemperatur gerührt und die Reaktionsmischung wird unter vermindertem Druck eingedampft. Man erhält 5 Gewichtsteile (99% d.Th.) des Natriumsalzes von 1-[3′-(2″-Chlor-4″-trifluormethylphenoxy)-6′-nitrophenyl]-3-methyl-1,3,5-triazin-2,4,6-(1H,3H,5H)-trion (Verbindung Nr. 3) vom Schmelzpunkt 220 bis 225 °C.

Entsprechend den o.a. Beispielen werden die in der folgenden Tabelle aufgeführten Verbindungen hergestellt.

| Nr. | 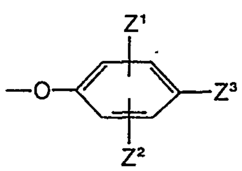 | Y | $R^2$ | $R^3$ | Fp [°C]/$n_D^{25}$/ Wellenlänge einer Bande im UR-Spektrum |
|---|---|---|---|---|---|
| 4 | 2-Chlor-4-trifluormethyl-phenyl | H | $CH_3$ | H | 100–105 |
| 5 | " | $NO_2$ | $C_2H_5$ | H | |
| 6 | " | " | " | $CH_3$ | |
| 7 | " | " | " | $Na^{\oplus}$ | |
| 8 | " | " | " | $C_2H_5$ | |
| 9 | " | " | $CH_3$ | $\overset{O}{\overset{\|}{C}}CH_3$ | |
| 10 | " | " | " | $\overset{O}{\overset{\|}{C}}C_6H_5$ | |
| 11 | " | " | " | $NH_4^{\oplus}$ | |
| 12 | " | " | 1-$C_3H_7$ | H | 218–220 |
| 13 | " | " | " | $CH_3$ | 150–155 |
| 14 | " | " | " | $Na^{\oplus}$ | |
| 15 | " | " | n-$C_6H_{13}$ | H | |
| 16 | " | " | " | $CH_3$ | |
| 17 | " | " | " | $Na^{\oplus}$ | |
| 18 | " | " | $CH_2$-$CH=CH_2$ | H | |
| 19 | " | " | " | $CH_3$ | |
| 20 | " | " | $CH_2$-$CH=CH_2$ | $Na^{\oplus}$ | |
| 21 | " | " | $CH_2CH_2Cl$ | H | |
| 22 | " | " | " | $CH_3$ | |

| Nr. | 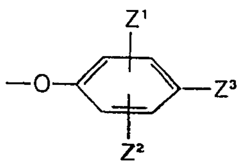 | Y | $R^2$ | $R^3$ | Fp [°C]/[$n_D^{25}$] Wellenlänge einer Bande im UR-Spektrum |
|---|---|---|---|---|---|
| 23 | 2-Chlor-4-trifluormethyl- | $NO_2$ | $CH_2CH_2Cl$ | $Na^{\oplus}$ | |
| 24 | phenyl | " | $CH_2CH_2F$ | H | |
| 25 | " | " | " | $CH_3$ | |
| 26 | " | " | " | $Na^{\oplus}$ | |
| 27 | " | " | $CH_2CH_2CN$ | H | |
| 28 | " | " | " | $CH_3$ | |
| 29 | " | " | " | $Na^{\oplus}$ | |
| 30 | " | " | $CH_2CH_2OH$ | H | |
| 31 | " | " | " | $CH_3$ | |
| 32 | " | " | " | $Na^{\oplus}$ | |
| 33 | " | " | $CH_2CH_2SH$ | H | |
| 34 | " | " | " | $CH_3$ | |
| 35 | " | " | " | $Na^{\oplus}$ | |
| 36 | " | " | $CH_2CH_2OCH_3$ | H | 170° Zers. |
| 37 | " | " | " | $CH_3$ | C=O 1680–1700 |
| 38 | " | " | " | $Na^{\oplus}$ | 110–114 |
| 39 | " | " | $CH_2CH_2OC_2H_5$ | H | |
| 40 | " | " | $CH(C_2H_5)CH_2OC_2H_5$ | H | |
| 41 | " | " | " | $CH_3$ | |
| 42 | " | " | " | $Na^{\oplus}$ | |
| 43 | " | " | $CH_2CH_2CH_2OC_4H_9$ | H | 100–110 |
| 44 | " | " | " | $CH_3$ | C=O 1690–1710 |
| 45 | " | " | " | $Na^{\oplus}$ | 100–105 |
| 46 | " | " | $CH_2CH_2CH_2OCH_2(CH_2)_{11}CH_3$ | H | |
| 47 | " | " | " | $CH_3$ | |
| 48 | " | " | " | $Na^{\oplus}$ | |
| 49 | " | " | $CH(CH_3)CH_2OCH_3$ | H | 200–205 |
| 50 | " | " | " | $CH_3$ | 60–65 |
| 51 | " | " | " | $Na^{\oplus}$ | 168–173 |
| 52 | " | " | $CH_2CH_2O$-n-$C_3H_7$ | H | |
| 53 | " | " | " | $CH_3$ | |
| 54 | " | " | " | $Na^{\oplus}$ | |
| 55 | " | " | $CH_2CH_2OCH_2CH_2OH$ | H | |
| 56 | " | " | " | $Na^{\oplus}$ | |
| 57 | " | " | $CH_2CH_2SCH_3$ | H | 170–174 |
| 58 | " | " | " | $CH_3$ | 60 |
| 59 | " | " | " | $Na^{\oplus}$ | |
| 60 | " | " | $CH_2CH_2S$-n-$C_8H_{17}$ | H | |
| 61 | " | " | " | $CH_3$ | |
| 62 | " | " | $CH(CH_3)CH_2SCH_3$ | H | |
| 63 | " | " | " | $CH_3$ | |
| 64 | " | " | " | $Na^{\oplus}$ | |
| 65 | " | " | $C(CH_3)_2CH_2SC_2H_5$ | H | |
| 66 | " | " | " | $CH_3$ | |
| 67 | " | " | $CH_2CH_2CH_2SCH_3$ | H | 105–110 |
| 68 | " | " | " | $CH_3$ | C=O 1690–1710 |
| 69 | " | " | " | $Na^{\oplus}$ | 115–120 |
| 70 | " | " | $CH_2CH_2N(CH_3)_2$ | H | |
| 71 | " | " | " | $CH_3$ | |
| 72 | " | " | " | $Na^{\oplus}$ | |
| 73 | " | " | 4-Chlorphenyl | H | |
| 74 | " | " | " | $CH_3$ | |
| 75 | " | " | 3,4-Dichlorphenyl | H | 95° Zers. |
| 76 | " | " | " | $CH_3$ | C=O 1690–1720 |
| 77 | " | " | 3-Trifluormethylphenyl | H | 145° Zers. |
| 78 | " | " | " | $CH_3$ | C=O 1690–1710 |

| Nr. | 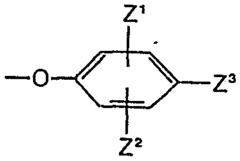 | Y | $R^2$ | $R^3$ | Fp [°C]/[$n_D^{25}$] Wellenlänge einer Bande im UR-Spektrum |
|---|---|---|---|---|---|
| 79 | 2-Chlor-4-trifluormethyl-phenyl | $NO_2$ | 4-Trifluormethoxyphenyl | H | |
| 80 | | " | " | $CH_3$ | |
| 81 | " | " | 3-Trifluormethylmercapto-phenyl | H | |
| 82 | " | " | " | $CH_3$ | |
| 83 | " | " | 4-Chlorbenzyl | H | |
| 84 | " | " | " | $CH_3$ | |
| 85 | " | " | 2,4-Dichlorbenzyl | H | |
| 86 | " | " | " | $CH_3$ | |
| 87 | " | " | 4-Methylbenzyl | H | |
| 88 | " | " | " | $CH_3$ | |
| 89 | " | " | Cyclopentyl | H | 160–166 |
| 90 | " | " | " | $CH_3$ | C=O 1700–1710 |
| 91 | " | " | " | $Na^{\oplus}$ | 125–130 |
| 92 | " | " | Cyclohexyl | H | 176–182 |
| 93 | " | " | Cyclohexyl | $CH_3$ | 60–65 |
| 94 | " | " | " | $Na^{\oplus}$ | 131–136 |
| 95 | " | Br | $CH_3$ | H | |
| 96 | " | " | " | $CH_3$ | |
| 97 | " | " | " | $Na^{\oplus}$ | |
| 98 | " | " | $CH_2CH_2OCH_3$ | H | |
| 99 | " | " | " | $CH_3$ | |
| 100 | " | " | " | $Na^{\oplus}$ | |
| 101 | " | CN | $CH_3$ | H | |
| 102 | " | " | " | $CH_3$ | |
| 103 | 2-Brom-4-trifluormethyl-phenyl | $NO_2$ | $CH_3$ | H | |
| 104 | " | " | " | $CH_3$ | |
| 105 | " | " | " | $Na^{\oplus}$ | |
| 106 | 2,6-Dichlor-4-trifluor-methylphenyl | " | " | H | |
| 107 | " | " | " | $CH_3$ | |
| 108 | " | " | " | $Na^{\oplus}$ | |
| 109 | 2-Chlor-4-trifluormeth-oxy-phenyl | " | " | H | |
| 110 | " | " | " | $CH_3$ | |
| 111 | 2-Chlor-4-trifluormethyl-mercapto-phenyl | " | " | H | |
| 112 | " | " | " | $CH_3$ | |
| 113 | 2.4-Dichlorphenyl | " | " | H | 224–229 |
| 114 | " | " | " | $CH_3$ | |
| 115 | 2,6-Dibromphenyl | " | " | H | 223–225 |
| 116 | " | " | " | $CH_3$ | |
| 117 | " | " | " | $Na^{\oplus}$ | |
| 118 | 2,4,6-Trichlorphenyl | " | " | H | |
| 119 | 3-Chlor-4-trifluormethyl-phenyl | " | " | H | |
| 120 | " | " | $CH_2CH_2OCH_3$ | H | |
| 121 | " | " | " | $CH_3$ | |
| 122 | 2-Brom-4-trifluormethyl-phenyl | " | " | H | 170–175 |
| 123 | " | " | " | $CH_3$ | 1,5403 |
| 124 | " | " | " | $Na^{\oplus}$ | 135–145 |
| 125 | 2-Chlor-4-fluorphenyl | $NO_2$ | $CH_2CH_2OCH_3$ | H | |
| 126 | " | " | " | $CH_3$ | |
| 127 | 2-Chlor-4-methylphenyl | " | " | H | |

| Nr. | 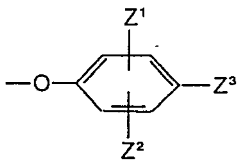 | Y | $R^2$ | $R^3$ | Fp [°C]/[$n_D^{25}$] Wellenlänge einer Bande im UR-Spektrum |
|---|---|---|---|---|---|
| 128 | 2-Chlor-4-methylpheny | $NO_2$ | $CH_2CH_2OCH_3$ | $CH_3$ | |
| 129 | 2-Chlor-4-trifluormethyl-phenyl | " | $CH_2CH_2S$-$C_6H_5$ | H | 217–222 |
| 130 | " | " | " | $CH_3$ | 55–61 |
| 131 | " | " | " | $Na^{\oplus}$ | 111–125 |
| 132 | " | " | " | $C_2H_5$ | 1,5732 |
| 133 | " | " | 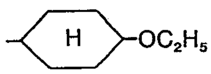 | H | 155 Zers. |
| 134 | " | " | " | $CH_3$ | 1,5146 |
| 135 | " | " | " | $C_2H_5$ | 1,5385 |
| 136 | " | " | " | $Na^{\oplus}$ | 140 Zers. |
| 137 | " | " | $CH_2CH_2OCH_3$ | $C_2H_5$ | 122–126 |
| 138 | " | " | $(CH_2)_3OC_4H_9$ | $C_2H_5$ | 1,5325 |
| 139 | " | " | Cyclopentyl | $C_2H_5$ | 1,5468 |
| 140 | " | " | Cyclohexyl | $C_2H_5$ | 1,5298 |
| 141 | " | " | $(CH_2)_3S$-$CH_3$ | $C_2H_5$ | 1,5597 |
| 142 | 2-Brom-4-trifluormethyl-phenyl | " | $CH_2CH_2OCH_3$ | $C_2H_5$ | |
| 143 | 2,4-Dichlorphenyl | " | " | H | C=O 1700–1710 |
| 144 | " | " | " | $CH_3$ | C=O 1700–1715 |
| 145 | " | " | " | $C_2H_5$ | 1,5744 |
| 146 | " | " | " | $Na^{\oplus}$ | 122–128 |
| 147 | 2,4-Dibromphenyl | " | " | H | 84–90 |
| 148 | " | " | " | $CH_3$ | 1,5631 |

Die Verbindungen der Formel I bzw. die sie enthaltenden herbiziden Mittel können beispielsweise in Form von direkt versprühbaren Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Giessen angewendet werden. Die Anwendungsformen richten sich ganz nach den Verwendungszwecken; sie sollten in jedem Fall möglichst die feinste Verteilung der erfindungsgemässen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wässrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe können Alkali-, Erdalkali-, Ammoniumsalze von
Ligninsulfonsäure, Naphthalinsulfonsäure,
Phenolsulfonsäure, Alkylarylsulfonate,
Alkylsulfate, Alkylsulfonate, Alkali- und
Erdalkalisalze der
Dibutylnaphthalinsulfonsäure,
Laurylethersulfat, Fettalkoholsulfate,
fettsaure Alkali- und Erdalkalisalze,
Salze sulfatierter Hexadecanole, Heptadecanole,
Octadecanole, Salze von sulfatiertem
Fettalkoholglykolether,
Kondensationsprodukte von sulfoniertem Naphthalin und Naphthalinderivaten mit Formaldehyd,
Kondensationsprodukte des Naphthalins bzw. der
Naphthalinsulfonsäuren mit
Phenol und Formaldehyd,
Polyoxyethylenoctylphenolehter,

ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose verwendet werden.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nussschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile des Wirkstoffs Nr. 1 mit 10 Gewichtsteilen N-Methyl-α-pyrrolidon und erhält eine Lösung, die zur Anwendung in Form kleinster Tropfen geeignet ist.

II. 20 Gewichtsteile des Wirkstoffs Nr. 38 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgiessen und feines Verteilen der Lösung in 100 000 Gewichtsteilen Wasser erhält man eine wässrige Dispersion, die 0,02 Gewichtsprozent des Wirkstoffs enthält.

III. 20 Gewichtsteile des Wirkstoffs Nr. 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 gewichtsprozent des Wirkstoffs enthält.

IV. 20 Gewichtsteile des Wirkstoffs Nr. 3 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingiessen und feines Verteilen der Lösung in 100 000 Gewichtsteilen Wasser erhält man eine wässrige Dispersion, die 0,02 Gewichtsprozent des Wirkstoffs enthält.

V. 80 Gewichtsteile des Wirkstoffs Nr. 37 werden mit 3 Gewichtsteilen des Natriumsalzes der Diisobutylnaphthalin-α-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 3 Gewichtsteile des Wirkstoffs Nr. 36 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 146 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines parafinischen Mineralöls vermischt. Man erhält eine stabile ölige Dispersion.

Die Applikation der Wirkstoffe bzw. der Mittel kann im Vorauflaufverfahren oder bei Nachauflaufanwendung erfolgen. Sind die Wirkstoffe für die Kulturpflanze weniger verträglich, so können auch Ausbringungstechniken angewandt werden. bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zierpflanzen und Wachstumsstatium 0,025 bis 10 kg/ha und mehr, vorzugsweise 0,1 bis 4,0 kg/ha, wobei sich die höheren Dosen besonders zur totalen Bekämpfung von Vegetationen eignen.

Die herbizide Wirkung der Verbindungen der Formel I bzw. der sie enthaltenden herbiziden Mittel wird durch Gewächshausversuche gezeigt:

Als Kulturgefässe dienen Plastikblumentöpfe mit 300 $cm^3$ Inhalt und lehmigem Sand mit etwa 1,5% Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Nach dem Aufbringen der Mittel beregnet man die Gefässe leicht, um Keimung und Wachstum in Gang zu bringen. Danach deckt man die Gefässe mit durchsichtigen Plastikhauben ab, die die Pflanzen angewachsen sind. Diese Abdeckung bewirkt ein

gleichmässiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die für die Nachauflaufanwendung benutzten Reispflanzen zieht man in einem mit Torfmull (peat) angereichertem Substrat an. Auch bei den Sojabohnen gibt man etwas Torfmull zu, um ein günstigeres Wachstum zu gewährleisten als in der oben beschriebenen Erde. Zur Nachauflaufbehandlung werden entweder direkt gesäte und in den gleichen Gefässen aufgewachsene Pflanzen ausgewählt, oder sie werden erst als Keimpflanzen getrennt angezogen und einige Tage vor der Behandlung in die Versuchsgefässe verpflanzt. Bei Nachauflaufbehandlung unterbleibt die Abdeckung.

Die Versuchsgefässe werden im Gewächshaus aufgestellt, wobei für wärmeliebende Arten wärmere Bereiche (20 bis 35 °C) und für solche gemässigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Bei den Testpflanzen handelt es sich um Abutilon theophrasti (Chinesischer Hanf), Amaranthus spp. (Fuchsschwanz-Arten), Arachys hypogaea (Erdnüsse), Chenopodium album (Weisser Gänsefuss), Datura stramonium (gemeiner Stechapfel), Echinochloa crus-galli (Hühnerhirse), Galeopsis tetrahit (gemeiner Holzzahn), Glycine max. (Sojabohnen), Oryza sativa (Reis), Sesbania exaltata (Turibaum), Sida spinosa, Sinapis alba (weisser Senf), Solanum nigrum (schwarzer Nachtschatten), Triticum aestivum (Weizen), Zea mays (Mais).

Vergleichsmittel ist die bekannte Verbindung 1-[4′-(2″-Chlor-4″-trifluormethylphenoxy)-phenyl]-3-methyl-1,3,4-triazin-2,4,6-trion (DE-OS 2 246 109).

Die Ergebnisse der Gewächshausversuche zeigen, dass beispielsweise die Verbindungen Nr. 1, 36 oder 37 bei Vorauflaufanwendung von beispielsweise 3,0 kg Wirkstoff/ha eine gute herbizide Wirkung aufweisen.

Bei der Prüfung auf selektive herbizide Eigenschaften bei Nachauflaufanwendung erweist sich die Verbindung Nr. 4 mit 0,5 kg Wirkstoff/ha als besser wirksam gegen das breitblättrige Beispielsunkraut Chenopodium als das Vergleichsmittel, ohne Sojabohnen- oder Maispflanzen zu schädigen.

Ebenso bekämpfen beispielsweise die Verbindungen Nr. 1, 3, 36, 37, 38, 44, 45, 69, 90, 93, 146 oder 148 bis 0,06, 0,125, 0,25 bzw. 0,5 kg Wirkstoff/ha im Nachauflaufverfahren eine Reihe breitblättriger unerwünschter Pflanzen, je nach Wirkstoff in Kulturen wie Reis, Weizen, Erdnüsse.

In Anbetracht der Verträglichkeit und der Vielseitigkeit der Applikationsmethoden können die erfindungsgemässen Verbindungen noch in einer weiteren grossen Zahl von Kulturpflanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuss |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weisse Rübe |
| Brassica rapa var. silvestris | Rübsen |
| Camellia sinensis | Teestrauch |
| Carthamus tinctorius | Saflor – Färberdistel |
| Carya illinoinensis | Pekannussbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |

| Botanischer Name | Deutscher Name |
|---|---|
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süsskartoffeln |
| Juglans regia | Walnussbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weisstanne |
| Pinus spp. | Kiefer |
| Pisum sativum | Gartenerbse |
| Prunus avium | Süsskirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preisselbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemässen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexan-1,3-dionderivate und andere in Betracht.

Ausserdem kann es von Nutzen sein, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Patentansprüche für die Vertragsstaaten BE, CH, DE, FR, GB, IT, LI, NL, SE**

1. 1,3,5-Triazinone der Formel

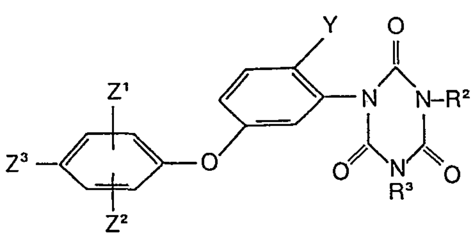

(I),

in der

$Z^1$ und $Z^2$ jeweils unabhängig voneinander Wasserstoff, Halogen, Nitro, Cyano, Carboxyl, Alkyl, Halogenalkyl oder Alkoxy mit jeweils bis zu 4 Kohlenstoffatomen,

$Z^3$ Halogen, Nitro, Cyano, Alkyl, Halogenalkyl, Alkoxy, Halogenalkoxy, Alkylmercapto, Halogenalkylmercapto, Alkylsulfinyl, Halogenalkylsulfinyl, Alkylsulfonyl oder Halogenalkylsulfonyl mit jeweils bis zu 4 Kohlenstoffatomen,

Y Wasserstoff, Halogen, Cyano oder Nitro,

$R^2$ Wasserstoff, einen gesättigten oder ungesättigten, unverzweigten oder verzweigten aliphatischen Kohlenwasserstoffrest mit bis zu 20 Kohlenstoffatomen, einen durch Halogen, Cyano, Hydroxy, Mercapto, Alkoxy oder Alkylmercapto mit jeweils bis zu 4 Kohlenstoffatomen, Phenylmercapto, Alkyl- oder Dialkylamino mit 1 bis 4 Kohlenstoffatomen in einer Alkylgruppe substituierten gesättigten, unverzweigten oder verzweigten aliphatischen Kohlenwasserstoffrest mit bis zu 10 Kohlenstoffatomen, einen gegebenenfalls durch Alkoxy mit 1 bis 4 Kohlenstoffatomen substituierten Cycloalkylrest mit 3 bis 7 Kohlenstoffatomen, einen gegebenenfalls durch Halogen, Alkyl oder Alkoxy mit jeweils 1 bis 4 Kohlenstoffatomen, Nitro, Cyano, Halogenalkyl, Halogenalkoxy oder Halogenalkylmercapto mit jeweils 1 bis 4 Kohlenstoffatomen substituierten Phenylrest oder einen gegebenenfalls durch Halogen substituierten Benzylrest und

$R^3$ Wasserstoff, Alkyl mit bis zu 4 Kohlenstoffatomen, gegebenenfalls halogensubstituiertes Acyl mit bis zu 7 Kohlenstoffatomen, oder – bei einfach negativ geladenem zugehörigem Triazinon-Stickstoffatom – ein Alkalimetallion oder ein gegebenenfalls alkyliertes Ammoniumion bedeuten.

2. 1,3,5-Triazinone der Formel I gemäss Anspruch 1, dadurch gekennzeichnet, dass $Z^1$ und $Z^2$ jeweils unabhängig voneinander Wasserstoffstoff, Chlor, Brom oder Cyano, $Z^3$ Chlor Brom, Methyl, Trifluormethoxy, Trifluormethylmercapto oder Trifluormethyl, Y Brom oder Nitro, $R^2$ Alkyl, Halogenalkyl, Cyanoalkyl, Alkoxy- oder Alkylmercaptoalkyl mit jeweils bis zu 4 Kohlenstoffatomen, Cycloalkyl mit 3 bis 7 Kohlenstoffatomen, durch Halogenalkyl mit 1 bis 4 Kohlenstoffatomen oder Halogen substituiertes Phenyl oder durch Halogen substituiertes Benzyl und $R^3$ Wasserstoff, Methyl oder Natrium bedeuten.

3. 1,3,5-Triazinone der Formel I gemäss Anspruch 1, dadurch gekennzeichnet, dass $Z^1$ und $Z^2$ jeweils unabhängig voneinander Wasserstoff, Chlor, Brom oder Cyano, $Z^3$ Chlor, Brom, Methyl, Trifluormethoxy, Trifluormethylmercapto oder Trifluormethyl, Y Nitro, $R^2$ Methyl, 2-Chlorethyl, 3,4-Dichlorphenyl oder 2-Methoxyethyl, und $R^3$ Wasserstoff, Methyl oder Natrium bedeuten.

4. 1-[3′-(2″-Chlor-4″-trifluormethylphenoxy)-6′-nitrophenyl]-3-methyl-1,3,5-triazin-2,4,5-(1H,3H,5H)-trion.

5. Verfahren zur Herstellung von 1,3,5-Triazinonen der Formel I gemäss Anspruch 1, dadurch gekennzeichnet, dass man einen phenoxysubstituierten Harnstoff der Formel

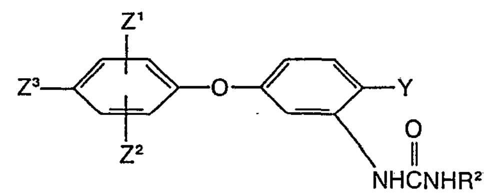

(II),

in der $Z^1$, $Z^2$, $Z^3$, Y und $R^2$ die im Anspruch 1 genannten Bedeutungen haben, mit einem substituierten Carbonylisocyanat der Formel

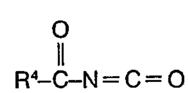

(III),

in der $R^4$ für Halogen, eine Alkoxygruppe oder eine Aryloxygruppe in einem inerten organischen Lösungsmittel, gegebenenfalls unter Zusatz eines Säureacceptors, bei Temperaturen zwischen −20 und +180 °C, zu einem 1,3,5-Triazinon der Formel

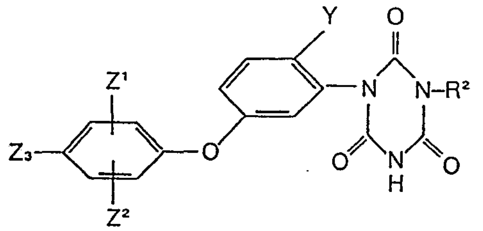,

in der $Z^1$, $Z^2$, $Z^3$, Y und $R^2$ die im Anspruch 1 genannten Bedeutungen haben, umsetzt und dieses dann gegebenenfalls mit einem Acylhalogenid der Formel $R^3COX$ oder einem Alkylhalogenid der Formel $R^3X$ oder einem Dialkylsulfat der Formel $(R^3O)_2SO_2$, wobei $R^3$ jeweils die im Anspruch 1 genannten Bedeutungen, ausgenommen Wasserstoff, hat und X für Halogen steht, acyliert oder alkyliert oder gegebenenfalls durch Umsetzung mit einem Alkalialkoholat, einem Alkalihydroxid oder einem gegenenfalls alkylierten Ammoniumhydroxid in ein Salz der Formel I überführt.

6. Herbizid, enthaltend ein 1,3,5-Triazinon der Formel I gemäss Anspruch 1.

7. Herbizid gemäss Anspruch 6, enthaltend inerte Zusatzstoffe.

8. Herbizid gemäss Anspruch 7, enthaltend ein 1,3,5-Triazinon gemäss Anspruch 2.

9. Herbizid gemäss Anspruch 7, enthaltend ein 1,3,5-Triazinon gemäss Anspruch 3.

10. Verfahren zur Bekämpfung unerwünschten Pflanzenwuchses, dadurch gekennzeichnet, dass man die unerwünschten Pflanzen und/oder die von unerwünschtem Pflanzenwuchs freizuhaltende Fläche mit einer herbizidwirksamen Menge eines 1,3,5-Triazinons der Formel I gemäss Anspruch 1 behandelt.

**Patentansprüche für den Vertragsstaat AT**

1. Herbizid, enthaltend inerte Zusatzstoffe und ein 1,3,5-Triazinon der Formel

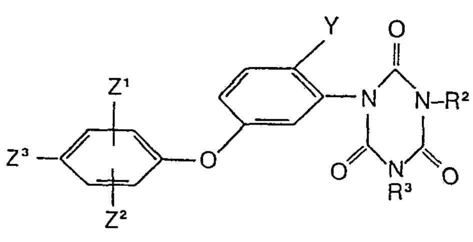 (I),

in der

$Z^1$ und $Z^2$ jeweils unabhängig voneinander Wasserstoff, Halogen, Nitro, Cyano, Carboxyl, Alkyl, Halogenalkyl oder Alkoxy mit jeweils bis zu 4 Kohlenstoffatomen,

$Z^3$ Halogen, Nitro, Cyano, Alkyl, Halogenalkyl, Alkoxy, Halogenalkoxy, Alkylmercapto, Halogenalkylmercapto, Alkylsulfinyl, Halogenalkylsulfinyl, Alkylsulfonyl oder Halogenalkylsulfonyl mit jeweils bis zu 4 Kohlenstoffatomen,

Y Wasserstoff, Halogen, Cyano oder Nitro,

$R^2$ Wasserstoff, einen gesättigten oder ungesättigten, unverzweigten oder verzweigten aliphatischen Kohlenwasserstoffrest mit bis zu 20 Kohlenstoffatomen, einen durch Halogen, Cyano, Hydroxy, Mercapto, Alkoxy oder Alkylmercapto mit jeweils bis zu 4 Kohlenstoffatomen, Phenylmercapto, Alkyl- oder Dialkylamino mit 1 bis 4 Kohlenstoffatomen in einer Alkylgruppe substituierten gesättigten, unverzweigten oder verzweigten aliphatischen Kohlenwasserstoffrest mit bis zu 10 Kohlenstoffatomen, einen gegebenenfalls durch Alkoxy mit 1 bis 4 Kohlenstoffatomen substituierten Cycloalkylrest mit 3 bis 7 Kohlenstoffatomen, einen gegebenenfalls durch Halogen, Alkyl oder Alkoxy mit jeweils 1 bis 4 Kohlenstoffatomen, Nitro, Cyano, Halogenalkyl, Halogenalkoxy oder Halogenalkylmercapto mit jeweils 1 bis 4 Kohlenstoffatomen substituierten Phenylrest oder einen gegebenenfalls durch Halogen substituierten Benzylrest und

$R^3$ Wasserstoff, Alkyl mit bis zu 4 Kohlenstoffatomen, gegebenenfalls halogensubstituiertes Acyl mit bis zu 7 Kohlenstoffatomen, oder – bei einfach negativ geladenem zugehörigem Triazinon-Stickstoffatom – ein Alkalimetallion oder ein gegebenenfalla alkyliertes Ammoniumion

bedeuten.

2. Herbizid, enthaltend inerte Zusatzstoffe und ein 1,3,5-Triazinon der Formel I gemäss Anspruch 1, wobei $Z^1$ und $Z^2$ jeweils unabhängig voneinander Wasserstoff, Chlor, Brom oder Cyano, $Z^3$ Chlor, Brom, Methyl, Trifluormethoxy, Trifluormethylmercapto oder Trifluormethyl, Y Brom oder Nitro, $R^2$ Alkyl, Halogenalkyl, Cyanoalkyl, Alkoxy- oder Alkylmercaptoalkyl mit jeweils bis zu 4 Kohlenstoffatomen, Cycloalkyl mit 3 bis 7 Kohlenstoffatomen, durch Halogenalkyl mit 1 bis 4 Kohlenstoffatomen oder Halogen substituiertes Phenyl oder durch Halogen substituiertes Benzyl und $R^3$ Wasserstoff, Methyl oder Natrium bedeuten.

3. Herbizid, enthaltend inerte Zusatzstoffe und ein 1,3,5-Triazinon der Formel I gemäss Anspruch 1, wobei $Z^1$ und $Z^2$ jeweils unabhängig voneinander Wasserstoff, Chlor, Brom oder Cyano, $Z^3$ Chlor, Brom, Methyl, Trifluormethoxy, Trifluormethylmercapto oder Trifluormethyl, Y Nitro, $R^2$ Methyl, 2-Chlorethyl, 3,4-Dichlorphenyl oder 2-Methoxyethyl und $R^3$ Wasserstoff, Methyl oder Natrium bedeuten.

4. Verfahren zur Herstellung von 1,3,5-Triazinonen der Formel I gemäss Anspruch 1, dadurch gekennzeichnet, dass man einen phenoxysubstituierten Harnstoff der Formel

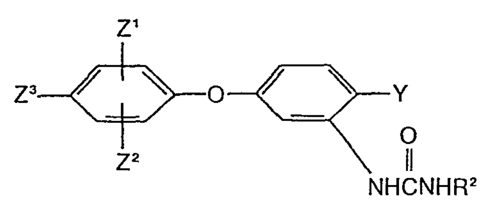 (II),

in der $Z^1$, $Z^2$, $Z^3$, Y und $R^2$ die im Anspruch 1 genannten Bedeutungen haben, mit einem substituierten Carbonylisocyanat der Formel

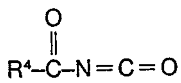
(III),

in der $R^4$ für Halogen, eine Alkoxygruppe oder eine Aryloxygruppe in einem inerten organischen Lösungsmittel, gegebenenfalls unter Zusatz eines Säureacceptors, bei Temperaturen zwischen −20 und +180 °C, zu einem 1,3,5-Triazinon der Formel

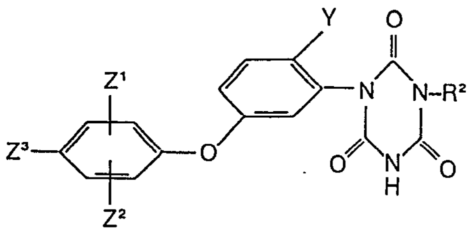
,

in der $Z^1$, $Z^2$, $Z^3$, Y und $R^2$ die im Anspruch 1 genannten Bedeutungen haben, umsetzt und dieses dann gegebenenfalls mit einem Acylhalogenid der Formel $R^3COX$ oder einem Alkylhalogenid der Formel $R^3X$ oder einem Dialkylsulfat der Formel $(R^3O)_2SO_2$, wobei $R^3$ jeweils die im Anspruch 1 genannten Bedeutungen, ausgenommen Wasserstoff, hat und X für Halogen steht, acyliert oder alkyliert oder gegebenenfalls durch Umsetzung mit einem Alkalialkoholat, einem Alkalihydroxid oder einem gegebenenfalls alkylierten Ammoniumhydroxid in ein Salz der Formel I überführt.

5. Verfahren zur Bekämpfung unerwünschten Pflanzenwuchses, dadurch gekennzeichnet, dass man die unerwünschten Pflanzen und/oder die von unerwünschtem Pflanzenwuchs freizuhaltende Fläche mit einer herbizid wirksamen Menge eines 1,3,5-Triazinons der Formel I gemäss Anspruch 1 behandelt.

**Claims for the contracting States: BE, CH, DE, FR, GB, IT, LI, NL, SE**

1. A 1,3,5-triazinone of the formula

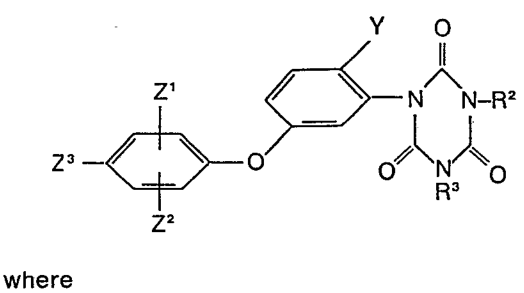
(I),

where

$Z^1$ and $Z^2$ independently of one another are hydrogen, halogen, nitro, cyano or carboxyl, or alkyl, haloalkyl or alkoxy, each of up to 4 carbon atoms,

$Z^3$ is halogen, nitro or cyano, or alkyl, haloalkyl, alkoxy, haloalkoxy, alkylmercapto, haloalkylmercapto, alkylsulfinyl, haloalkylsulfinyl, alkylsulfonyl or haloalkylsulfonyl, each of up to 4 carbon atoms,

Y is hydrogen, halogen, cyano or nitro,

$R^2$ is hydrogen or a saturated or unsaturated, traight-chain of branched aliphatic hydrocarbon radical of up to 20 carbon atoms, or $R^2$ is a saturated, straight-chain or branched aliphatic hydrocarbon radical of up to 10 carbon atoms which is substituted by halogen, cyano, hydroxyl or mercapto, or by alkoxy or alkylmercapto, each of up to 4 carbon atoms, or by phenylmercapto, or by alkylamino or dialkylamino, where each alkyl is of 1 to 4 carbon atoms, or $R^2$ is cycloalkyl of 3 to 7 carbon atoms which is unsubstituted or substituted by alkoxy of 1 to 4 carbon atoms, or $R^2$ is phenyl which is unsubstituted or substituted by halogen, or by alkyl or alkoxy, each of 1 to 4 carbon atoms, or by nitro or cyano, or by haloalkyl, haloalkoxy or haloalkylmercapto, each of 1 to 4 carbon atoms, or $R^2$ is benzyl or halobenzyl, and

$R^3$ is hydrogen, alkyl of up to 4 carbon atoms, acyl of up to 7 carbon atoms which is unsubstituted or substituted by halogen, or is, when the relevant nitrogen atom of the triazinone has a single negative charge, an alkali metal ion or an optionally alkylated ammonium ion.

2. A 1,3,5-triazinone of the formula I as claimed in claim 1, where $Z^1$ and $Z^2$ independently of one another are hydrogen, chlorine, bromine or cyano, $Z^3$ ist chlorine, bromine, methyl, trifluoromethoxy, trifluoromethylmercapto or trifluoromethyl, Y is bromine or nitro, $R^2$ is alkyl, haloalkyl, cyanoalkyl, alkoxy- or alkylmercaptoalkyl, each of up to 4 carbon atoms, cycloalkyl of 3 to 7 carbon atoms, phenyl substituted by haloalkyl of 1 to 4 carbon atoms or by halogen, or benzyl substituted by halogen, and $R^3$ is hydrogen, methyl or sodium.

3. A 1,3,5-triazinone of the formula I as claimed in claim 1, where $Z^1$ and $Z^2$ independently of one another are hydrogen, chlorine, bromine or cyano, $Z^3$ is chlorine, bromine, methyl, trifluoromethoxy, trifluoromethylmercapto or trifluoromethyl, Y is nitro, $R^2$ is methyl, 2-chloroethyl, 3,4-dichlorophenyl or 2-methoxyethyl, and $R^3$ is hydrogen, methyl or sodium.

4. 1-[3′-(2″-Chloro-4″-trifluoromethylphenoxy)-6′-nitrophenyl]-3-methyl-1,3,5-triazine-2,4,6-(1H,3H,5H)-trione.

5. A process for the preparation of a 1,3,5-triazinone of the formula I as claimed in claim 1, wherein a phenoxysubstituted urea of the formula

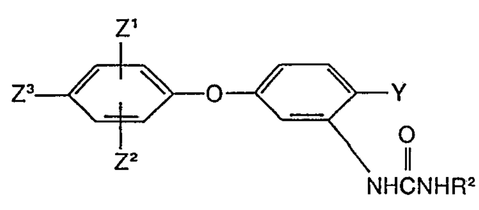
(II),

where $Z^1$, $Z^2$, $Z^3$, Y and $R^2$ have the meanings given in claim 1, is reacted with a substituted carbonyl isocyanate of the formula

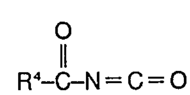
(III),

where $R^4$ is halogen, alkoxy or aryloxy, in an inert organic solvent, with or without the addition of an acid acceptor, at from −20 to +180 °C, to give a substituted 1,3,5-triazinone of the formula

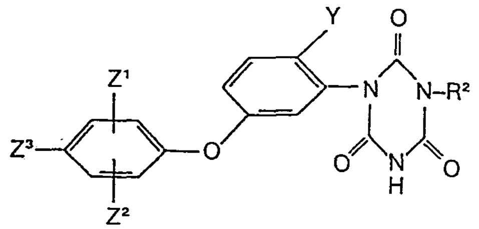
,

where $Z^1$, $Z^2$, $Z^3$, Y and $R^2$ have the meanings given in claim 1, and, if desired, this product can then be acylated or alkylated with an acyl halide of the formula $R^3COX$, an alkyl halide of the formula $R^3X$ or a dialkylsulfate of the formula $(R^3O)_2SO_2$, where $R^3$ in each case has the meanings given in claim 1, with the exception of hydrogen, and X is halogen, or converted into a salt of the formula I by reaction with an alkali metal alcoholate, an alkali metal hydroxide or an optionally alkylated ammonium hydroxide.

6. A herbicide containing a 1,3,5-triazinone of the formula I as claimed in claim I.

7. A herbicide as claimed in claim 6, containing inert additives.

8. A herbicide as claimed in claim 7, containing a 1,3,5-triazinone of the formula I as claimed in claim 2.

9. A herbicide as claimed in claim 7, containing a 1,3,5-triazinone of the formula I as claimed in claim 3.

10. A process for controlling unwanted plant growth, wherein the unwanted plants and/or the area to be kept free from unwanted plant growth are treated with a herbicidally effective amount of a 1,3,5-triazinone of the formula I as claimed in claim 1.

**Claims for the contracting State: AT**

1. A herbicide containing inert additives and a 1,3,5-triazinone of the formula

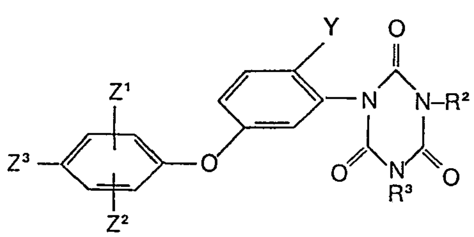
(I),

where

$Z^1$ and $Z^2$ independently of one another are hydrogen, halogen, nitro, cyano or carboxyl, or alkyl, haloalkyl or alkoxy, each of up to 4 carbon atoms,

$Z^3$ is halogen, nitro or cyano, or alkyl, haloalkyl, alkoxy, haloalkoxy, alkylmercapto, haloalkylmercapto, alkylsulfinyl, haloalkylsulfinyl, alkylsulfonyl or haloalkylsulfonyl, each of up to 4 carbon atoms,

Y is hydrogen, halogen, cyano or nitro,

$R^2$ is hydrogen or a saturated or unsaturated straight-chain or a branched aliphatic hydrocarbon radical of up to 20 carbon atoms, or $R^2$ is a saturated, straight-chain or branched aliphatic hydrocarbon radical of up to 10 carbon atoms which is substituted by halogen, cyano, hydroxyl or mercapto, or by alkoxy or alkylmercapto, each of up to 4 carbon atoms, or by phenylmercapto, or by alkylamino or dialkylamino, where each alkyl is of 1 to 4 carbon atoms, or $R^2$ is cycloalkyl of 3 to 7 carbon atoms which is unsubstituted or substituted by alkoxy of 1 to 4 carbon atoms, or $R^2$ is phenyl which is unsubstituted or substituted by halogen, or by alkyl or alkoxy, each of 1 to 4 carbon atoms, or by nitro or cyano, or by haloalkyl, haloalkoxy or haloalkylmercapto, each of 1 to 4 carbon atoms, or $R^2$ is benzyl or halobenzyl, and

$R^3$ is hydrogen, alkyl of up to 4 carbon atoms, acyl of up to 7 carbon atoms which is unsubstituted or substituted by halogen, or is, when the relevant nitrogen atom of the triazinone has a single negative charge, an alkali metal ion or an optionally alkylated ammonium ion.

2. A herbicide containing inert additives and a 1,3,5-triazinone of the formula I as claimed in claim 1, where $Z^1$ and $Z^2$ independently of one another are hydrogen, chlorine, bromine or cyano, $Z^3$ is chlorine, bromine, methyl, trifluoromethoxy, trifluoromethylmercapto or trifluoromethyl, Y is bromine or nitro, $R^2$ is alkyl, haloalkyl, cyanoalkyl, alkoxy- or alkylmercaptoalkyl, each of up to 4 carbon atoms, cycloalkyl of 3 to 7 carbon atoms, phenyl substituted by haloalkyl of 1 to 4 carbon atoms or by halogen, or benzyl substituted by halogen, and $R^3$ is hydrogen, methyl or sodium.

3. A herbicide containing inert additives and a 1,3,5-triazinone of the formula I as claimed in claim 1, where $Z^1$ and $Z^2$ independently of one another are hydrogen, chlorine, bromine or cyano, $Z^3$ is chlorine, bromine, methyl, trifluoromethoxy, trifluoromethylmercapto or trifluoromethyl, Y is nitro, $R^2$ is methyl, 2-chloroethyl, 3,4-dichlorophenyl or 2-methoxyethyl, and $R^3$ is hydrogen, methyl or sodium.

4. A process for the preparation of a 1,3,5-triazinone of the formula I as claimed in claim 1, wherein a phenoxysubstituted urea of the formula

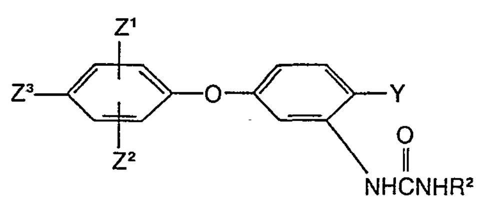
(II),

where $Z^1$, $Z^2$, $Z^3$, Y and $R^2$ have the meanings given in claim 1, is reacted with a substituted carbonyl isocyanate of the formula

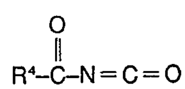
(III),

where $R^4$ is halogen, alkoxy or aryloxy, in an inert organic solvent, with or without the addition of an acid acceptor, at from −20 to +180 °C, to give a substituted 1,3,5-triazinone of the formula

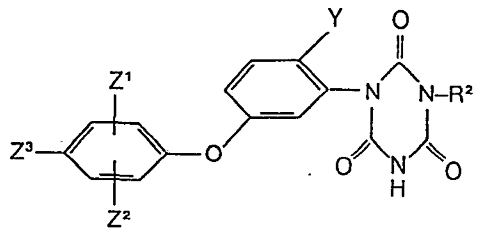

,

where $Z^1$, $Z^2$, $Z^3$, Y and $R^2$ have the meanings given in claim 1, and, if desired, this product can then be acylated or alkylated with an acyl halide of the formula $R^3COX$, an alkyl halide of the formula $R^3X$ or a dialkylsulfate of the formula $(R^3O)_2SO_2$, where $R^3$ in each case has the meanings given in claim 1, with the exception of hydrogen, and X is halogen, or converted into a salt of the formula I by reaction with an alkali metal alcoholate, an alkali metal hydroxide or an optionally alkylated ammonium hydroxide.

5. A process for controlling unwanted plant growth, wherein the unwanted plants and/or the area to be kept free from unwanted plant growth are treated with a herbicidally effective amount of a 1,3,5-triazinone of the formula I as claimed in claim 1.

**Revendications pour les Etats contractants: BE, CH, DE, FR, GB, IT, LI, NL, SE**

1. 1,3,5-triazinones de formule

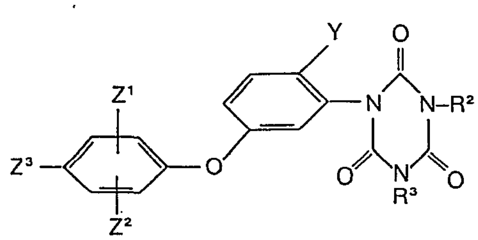

(I)

dans laquelle

$Z_1$ et $Z^2$ représentent chacun indépendamment l'un de l'autre, hydrogène, halogène, nitro, cyano, carboxyle, alkyle, halogènalkyle ou alcoxy ayant chacun jusqu'à 4 atomes de carbone,

$Z^3$ halogène, nitro, cyano, alkyle, halogènalkyle, alcoxy, halogènalcoxy, alkylmercapto, halogènalkylmercapto, alkylsulfinyle, halogènalkylsulfinyle, alkylsulfonyle, ou halogènalkylsulfonyle ayant chacun jusqu'à 4 atomes de carbone,

Y représente hydrogène, halogène, cyano ou nitro,

$R^2$ représente hydrogène, un reste d'hydrocarbure aliphatique, saturé ou non saturé, ramifié ou non ramifié, ayant jusqu'à 20 atomes de carbone, un reste d'hydrocarbure aliphatique, ayant jusqu'à 10 atomes de carbone, saturé ou non saturé, ramifié ou non ramifié, substitué par halogène, cyano, hydroxy, mercapto, alcoxy ou alkylmercapto ayant chacun jusqu'à 4 atomes de carbone, phénylmercapto, alkyl ou dialkylamino ayant chacun de 1 à 4 atomes de carbone dans un groupe alkyle, un reste cycloalkyle de 3 à 7 atomes de carbone, éventuellement substitué par alcoxy de 1 à 4 atomes de carbone, un reste phényle, éventuellement substitué par halogène, alkyle ou alcoxy ayant chacun 1 à 4 atomes de carbone, nitro, cyano, halogènalkyle, halogènalcoxy ou halogènalkylmercapto ayant chacun 1 à 4 atomes de carbone, ou un reste benzyle éventuellement substitué par halogène et,

$R^3$ représente hydrogène, alkyle ayant jusqu'à 4 atomes de carbone, acyle ayant jusqu'à 7 atomes de carbone, éventuellement halogèno-substitué, ou – dans le cas de l'atome d'azote de triazinone correspondant, chargé une fois négativement – un ion métal alcalin ou un ion ammonium éventuellement alkylé.

2. 1,3,5-triazinones de formule I, selon la revendication 1, caractérisées par le fait que $Z^1$ et $Z^2$ représentent chacun, indépendamment l'un de l'autre, hydrogène, chlore, brome ou cyano, $Z^3$ chlore, brome, méthyle, trifluorométhoxy, trifluorométhylmercapto ou trifluorométhyle, Y brome ou nitro, $R^2$ alkyle, halogènalkyle, cyanoalkyle, alcoxy- ou alkylmercapto-alkyle, ayant chacun jusqu'à 4 atomes de carbone, cycloalkyle ayant 3 à 7 atomes de carbone, phényle substitué par halogènalkyle de 1 à 4 atomes de carbone ou par halogène, ou benzyle substitué par halogène, et $R^3$, hydrogène, méthyle ou sodium.

3. 1,3,5-triazinones de formule I selon la revendication 1, caractérisées par le fait que $Z^1$ et $Z^2$ représentent chacun, indépendamment l'un de l'autre, hydrogène, chlore, brome ou cyano, $Z^3$ chlore, brome, méthyle, trifluorométhoxy, trifluorométhylmercapto ou trifluorométhyle, Y nitro $R^2$ méthyle, 2-chloréthyle, 3,4-dichlorophényle, ou 2-méthoxyéthyle, et $R^3$ hydrogène, méthyle ou sodium.

4. 1-[3'-(2''-Chloro-4''-trifluorométhyl-phénoxy)-6'-nitro-phényl]-3-méthyl-1,3,5-triazin-2,4,6-(1H,3H,5H)-trione.

5. Procédé de préparation de 1,3,5-triazinones de formule I selon la revendication 1, caractérisé par le fait qu'on fait réagir, éventuellement avec addition d'un accepteur d'acide, à des températures comprises entre −20 et +180 °C une urée phénoxy-substituée de formule

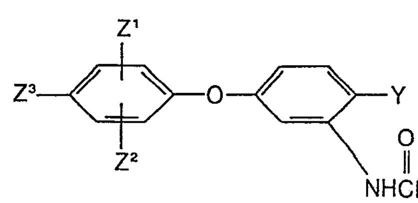

(II),

dans laquelle $Z^1$, $Z^2$, $Z^3$, Y et $R^2$ ont les significations indiquées dans la revendication 1, avec un isocyanate de carbonyle substitué de formule

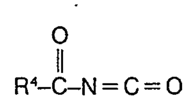

(III),

dans laquelle $R^4$ représente halogène, un groupe alcoxy ou un groupe aryloxy, pour obtenir une 1,3,5-triazinone de formule

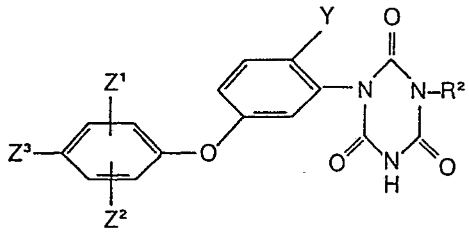

,

dans laquelle $Z^1$, $Z^2$, $Z^3$, Y et $R^2$ ont les significations indiquées dans la revendication 1, et on acyle ou alkyle cette triazonine, éventuellement, avec un halogénure d'acyle de formule $R^3COX$ ou un halogénure d'alkyle de formule $R^3X$ ou un sulfate de dialkyle de formule $(R^3O)_2SO_2$, $R^3$ ayant la signification indiquée dans la revendication 1, excepté hydrogène, et X étant mis pour halogène, ou on transforme cette triazinone en un sel de formule I par réaction avec un alcoolate alcalin, un hydroxyde alcalin ou hydroxyde d'ammonium éventuellement alkylé.

6. Herbicide contenant une 1,3,5-triazinone de formule I selon la revendication 1.

7. Herbicide selon la revendication 6 contenant une matière additive inerte.

8. Herbicide selon la revendication 7, contenant und 1,3,5-triazinone selon la revendication 2.

9. Herbicide selon la revendication 7, contenant une 1,3,5-triazinone selon la revendication 3.

10. Procédé pour lutter contre la croissance de plantes indésirables, caractérisé par le fait que l'on traite les plantes indésirables et/ou les surfaces à maintenir exemptes de croissance de plantes indésirables avec une quantité efficace au point de vue herbicide d'une 1,3,5-triazinone de formule I selon la revendication 1.

**Revendications pour l'Etat contractant: AT**

1. Herbicide contenant une matière additive inerte et une 1,3,5-triazinone de formule

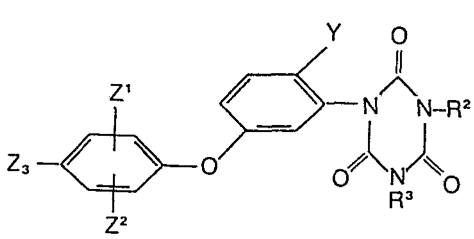

(I)

dans laquelle

$Z^1$ et $Z^2$ représentent chacun, indépendamment l'un de l'autre, hydrogène, halogène, nitro, cyano, carboxyle, alkyle, halogènalkyle ou alcoxy ayant chacun jusqu'à 4 atomes de carbone,

$Z^3$ halogène, nitro, cyano, alkyle, halogènalkyle, alcoxy, halogènalcoxy, alkylmercapto, halogènalkylmercapto, alkylsulfinyle, halogènalkylsulfinyle, alkylsulfonyle, ou halogènalkylsulfonyle ayant chacun jusqu'à 4 atomes de carbone,

Y représente hydrogène, halogène, cyano ou nitro,

$R^2$ représente hydrogène, un reste d'hydrocarbure aliphatique, saturé ou non saturé, ramifié ou non ramifié, ayant jusqu'à 20 atomes de carbone, un reste d'hydrocarbure aliphatique, ayant jusqu'à 10 atomes de carbone, saturé ou non saturé, ramifié ou non ramifié, substitué par halogène, cyano, hydroxy, mercapto, alcoxy ou alkylmercapto ayant chacun jusqu'à 4 atomes de carbone, phénylmercapto, alkyl ou dialkylamino ayant chacun de 1 à 4 atomes de carbone dans un groupe alkyle, un reste cycloalkyle de 3 à 7 atomes de carbone, éventuellement substitué par alcoxy ayant chacun 1 à 4 atomes de carbone, un reste phényle, éventuellement substitué par halogène, alkyle ou alcoxy ayant chacun 1 à 4 atomes de carbone, nitro, cyano, halogènalkyle, halogènalcoxy ou halogènalkylmercapto ayant chacun 1 à 4 atomes de carbone, ou un reste benzyle éventuellement substitué par halogène et,

$R^3$ représente hydrogène, alkyle ayant jusqu'à 4 atomes de carbone, acyle ayant jusqu'à 7 atomes de carbone, èventuellement halogèno-substitué, ou – dans le cas de l'atome d'azote de triazinone correspondant, chargé une fois négativement – un ion métal alcalin ou un ion ammonium éventuellement alkylé.

2. Herbicide contenant une matière additive inerte et une 1,3,5-triazinone de formule I selon la revendication 1, dans lequel $Z^1$ et $Z^2$ représentent chacun, indépendamment l'un de l'autre, hydrogène, chlore, brome ou cyano, $Z^3$ chlore, brome, méthyle, trifluorométhoxy, trifluorométhylmercapto ou trifluorométhyle, Y brome ou nitro, $R^2$ alkyle, halogènalkyle, cyanoalkyle, alcoxy- ou alkylmercapto-alkyle, ayant chacun jusqu'à 4 atomes de carbone, cycloalkyle ayant 3 à 7 atomes de carbone, phényle substitué par halogènalkyle de 1 à 4 atomes de carbone ou par halogène, ou benzyle substitué par halogène, et $R^3$ hydrogène, méthyle ou sodium.

3. Herbicide contenant une matière additive inerte et une 1,3,5-triazinone de formule I selon la revendication 1, dans lequel $Z^1$ et $Z^2$ représentent chacun, indépendamment l'un de l'autre, hydrogène, chlore, brome ou cyano, $Z^3$ chlore, brome, méthyle, trifluorométhoxy, trifluorométhylmercapto ou trifluorométhyle, Y nitro, $R^2$ méthyle, 2-chloréthyle, 3,4-dichlorophényle, ou 2-méthoxyéthyle, et $R^3$ hydrogène, méthyle ou sodium.

4. Procédé de préparation de 1,3,5-triazinones de formule I selon la revendication 1, caractérisé par le fait qu'on fait réagir, éventuellement avec addition d'un accepteur d'acide, à des températures comprises entre −20 et +180 °C une urée phénoxy-substituée de formule

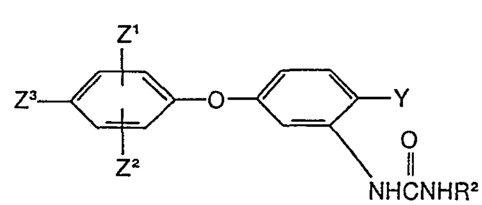

(II)

dans laquelle $Z^1$, $Z^2$, $Z^3$, Y et $R^2$ ont les significations indiquées dans la revendication 1, avec un isocyanate de carbonyle substitué de formule

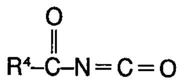

(III)

dans laquelle $R^4$ représente halogène, un groupe alcoxy ou un groupe aryloxy, pour obtenir une 1,3,5-triazinone de formule

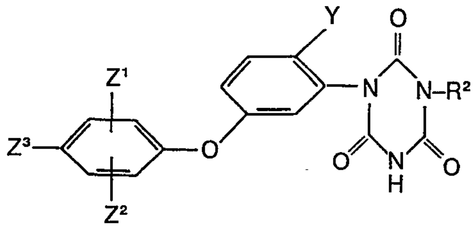

dans laquelle $Z^1$, $Z^2$, $Z^3$, Y et $R^2$ ont les significations indiquées dans la revendication 1, et on acyle ou alkyle cette triazonine, éventuellement, avec un halogénure d'acyle de formule $R^3COX$ ou un halogénure d'alkyle de formule $R^3X$ ou un sulfate de dialkyle de formule $(R^3O)_2SO_2$, $R^3$ ayant la signification indiquée dans la revendication 1, excepté hydrogène, et X étant mis pour halogène, ou on transforme cette triazinone en un sel de formule I par réaction avec un alcoolate alcalin, un hydroxyde alcalin ou hydroxyde d'ammonium éventuellement alkylé.

5. Procédé pour lutter contre la croissance de plantes indésirables, caractérisé par le fait que l'on traite les plantes indésirables et/ou les surfaces à maintenir exemptes de croissance de plantes indésirables avec une quantité efficace au point de vue herbicide d'une 1,3,5-triazinone de formule I selon la revendication 1.